# EUROPEAN PATENT APPLICATION

(11) **EP 1 159 915 A2**
(43) Date of publication of application: **05.12.2001**
(21) Application number: 01303582.9
(22) Date of filing: 19.04.2001
(51) Int. Cl.: A61B 5/026

(54) **Ultrasonic blood flow rate sensing**

(30) Priority: 19.04.2000 GB 0009742
(71) Applicant: Skidmore, Robert, Stoke Bishop, Bristol BS9 1TV (GB)
(72) Inventor: Skidmore, Robert, Stoke Bishop, Bristol BS9 1TV (GB)
(74) Representative: Harrison, Ivor Stanley

(57) **Abstract**

Pulsed ultrasound sensor 10, connected to means 11 for controlling its passage and positioning along and within an exogenous conduit 13 sited within a bodily tract 15 with access to the extracorporeal environment, transmits into a blood vessel 14 and times its acquisition of signals scattered from the blood flow F to probe two or more adjacent locations 16 and 18 within the blood vessel 14. Signals returned from the blood vessel (e.g. energy or Doppler shift due to blood flow F) can be monitored to align the transmission path of the sensor with the blood vessel axis (figures 4 and 5). The apparatus can be used to measure cardiovascular parameters in a non-invasive, non-surgical manner with minimal intervention from a physician.

## Description

This invention relates to apparatus and methods for sensing parameters, especially biological parameters. In particular, the invention relates to measuring blood flow rate in a blood vessel in a non-invasive manner.

It is known to transmit ultrasound into a blood vessel and measure the Doppler shift of ultrasound waves scattered by the blood flow. Taking the example of a red blood cell, waves scattered from the blood cell towards a receiver will experience a Doppler shift dependent upon the component of the velocity of the blood cell in the direction of the receiver. In other words, the velocity of scatterers in a blood flow can be deduced from the Doppler shift imparted to the scattered waves. If the size of the cross-section of the stream of flowing blood is known or can be deduced, then, given the flow velocity, the flow rate can be calculated. A knowledge of the rate of blood flow is desirable in many (e.g., medical) situations.

It is desirable to make accurate measurements of such flow rates. To increase the accuracy of a measurement made using a Doppler technique, it is desirable to measure flow velocity at different points across the blood flow to estimate the variation of flow velocity within the flow. Measurements of flow velocity at different locations across the flow can be made by pulsing the ultrasound transmitter. At a given instant in time, the signal acquired by the receiver corresponds to ultrasound waves being scattered from the present location of the transmitted pulse. Therefore, by determining a start and an end time for reception, i.e. by time-gating the received signal, the location from which the scattered ultrasound waves are received may be dictated. Several locations on the transmission path across the blood flow can be examined by "multi-gating" the received scattered ultrasound waves.

Having determined the velocity profile within the blood flow, an average flow velocity may be determined. To derive the flow rate, a knowledge of the cross sectional area of the flow is required. A conventional method of measuring this cross sectional area involves scanning a gate across the blood flow and seeking the points at which the Doppler shift of the received scattered ultrasound waves first appears and then eventually disappears.

It is generally desirable that biological parameter sensing devices such as those using Doppler ultrasound are placed in close proximity to the actual material whose parameters are to be determined. In the case of blood flow measurement, therefore, it is advantageous to have the transmitter and receiver placed on or near the exterior surface of the appropriate blood vessel. For measurements during surgical procedures, this rarely presents a significant problem. Pre- or post-surgically, however, the measurement of, for example, blood flow in the major central vessels using such sensing techniques is very difficult because of the surrounding structures and tissues which cause attenuation of and interference with the receivable signal of interest. Thus there is a need for a method of accurately determining biological parameters such as arterial or venous blood flow and blood vessel properties in a non-surgical, non-invasive manner.

It is an object of this invention to provide non-invasive sensing apparatuses and methods of accurately determining biological parameters without the need for surgical intervention.

According to one aspect, the invention provides a method of remotely locating biological material interfaces, the method comprising transmitting a signal into the biological material using a sensing apparatus located in an exogenous conduit placed within a bodily tract with access to the extracorporeal environment, detecting the signals returned from at least one pair of locations along the path of the transmitted signal and determining from the returned signals whether there is an interface between each pair of locations.

According to another, and related, aspect of the invention, there is provided apparatus for remotely locating biological material interfaces, the apparatus comprising means for transmitting a signal into the biological material, means for detecting the signals returned from at least one pair of locations along the path of the transmitted signal, means for determining from the returned signals whether there is an interface between each pair of locations, means for passing at least the transmission and detection means along an exogenous conduit placed within a bodily tract with access to the extracorporeal environment and means for remotely controlling the position of the transmission and detection means within the exogenous conduit.

The invention thus provides a method of locating a biological material interface, such as the boundary between a blood flow and an artery wall, which is capable of improved accuracy and which is extracorporeally controllable and capable of being applied to a subject without surgical intervention.

In a preferred embodiment, the detecting step comprises detecting signals from a plurality of pairs of locations along the transmission path so as to notionally sweep a pair of locations (or time-gates) along the path. Preferably, the locations in each pair abut one another. The selected pairs of locations may be chosen so that the notional sweeping action is an essentially continuous sweeping action.

Preferably, the transmission and detection means are capable both of being moved along the exogenous conduit and of being rotated within the exogenous conduit about a longitudinal axis of the exogenous conduit.

Advantageously, the determining step comprises comparing the returned signals on the basis of at least one of their energy and frequency. Preferably, the transmitted signal is an ultrasonic signal.

In a particularly preferred embodiment, the method comprises locating two biological material interfaces, and may involve determining their separation. In this way, it is possible to determine the boundaries of, for example, a fluid flow in a biological vessel. With knowledge of the boundaries of such a flow, the flow rate may be determined.

According to another aspect, the invention provides a method of remotely aligning a sensor relative to a biological vessel, comprising providing a sensor capable of being passed, using passing means, along an exogenous conduit placed within a bodily tract with access to the extracorporeal environment and which sensor transmits a signal along a path, remotely positioning the sensor such that the path intersects the biological vessel, detecting the signals returned from the biological vessel, and determining from the returned signals whether the path intersects substantially the centre of the biological vessel.

According to a further, and related, aspect, the invention also provides apparatus for remotely sensing a biological vessel, the apparatus comprising sensing means which transmits a signal along a path, detector means for detecting signals returned from the biological vessel, determining means for determining from the returned signals whether the transmission path intersects substantially the centre of the biological vessel, means for passing at least the transmission and detection means along an exogenous conduit placed within a bodily tract with access to the extracorporeal environment and means for remotely controlling the position of the transmission and detection means within the exogenous conduit.

Thus the invention facilitates the improvement of the non-surgical remote alignment of a sensor with a biological vessel to improve the accuracy of measurements made on the latter.

Advantageously, the signals returning from the biological vessel are detected for several different orientations of the sensor relative to the biological vessel. These detected signals may then be used to determine the location of the centre of the biological vessel relative to the sensor path and permit the sensor to be realigned so that the path substantially intersects the centre of the biological vessel..

In a preferred embodiment, signals returned from at least one location on the path are detected and the sensor is realigned until at least one property of the returned signals is optimised. In one case, signals returned from just one location on the path are detected and the sensor is realigned until, for at least one property, the value thereof for signals returned from said one location is optimised. In an alternative arrangement, signals returned from a plurality of locations on the path are detected and the sensor is realigned until, for at least one property, the total of the values thereof for signals returned from the locations is optimised.

In a preferred embodiment, the transmission means provides two paths for conveying signals between the sensing means and the processing means, the two paths being arranged such that they experience substantially the same interference and/or distortion. This provides that the value of the difference between the signals on the two paths is less effected by interference and/or distortion. In one embodiment, the transmission means may comprise a pair of twisted wires.

Advantageously, the sensor apparatus further comprises isolating means for preventing potentially damaging signals being conveyed between the processing means and the sensing means or the living body via the transmission means. This provides for the protection of the equipment and the test subject. Where the transmission means comprises said aforementioned two paths, the isolating means ideally comprises means for providing a signal indicative of the difference between the signals on the paths of the transmission means. For example, the isolating means may be a transformer, possibly of the single-turn, air-gap kind.

In the preferred embodiment, the sensing means comprises at least one piezoelectric transducer. It is particularly advantageous to employ dual transmission and detection transducers with a known, fixed angle between their signal paths. As is known in the art, this arrangement obviates the need to know the angle of incidence of the path with the biological vessel in order to calculate Doppler shift-generating parameters.

The means for passing the transmission and detection means along the exogenous conduit may comprise a flexible cable which is capable, in use, of being rotated about its longitudinal axis without developing substantial elastic twisting forces. The remote control of the position of the sensor within the exogenous conduit may be achieved by the use of a rotatable knob connected to the extracorporeal end of the passing means. The rotation of the knob causes rotation of the sensor within the exogenous conduit. Means, such as a potentiometer based resolver, may be used to measure the angular orientation of the sensor. This angular information can be used in displaying data derived from the detected returned signals (e.g. Blood flow velocities) in a desired format, for example, a swept radar-like plot.

In a preferred embodiment, the exogenous conduit is an oesophageal feeding tube, such as is commonly passed through the mouth or nose, along the oesophagus and into the stomach, and which is hereafter referred to simply as a nasogastric tube. In this preferred embodiment, the biological vessel under interrogation may be the descending aorta, in which case the biological material interface of interest would be the interior wall of the descending aorta, the sensor residing in the nasogastric tube at a depth where the oesophagus is in close proximity to the descending aorta.

Thus, the apparatus and method of this invention provide means for determining characteristics of cardiovascular function such as blood flow velocity, flow profile, vessel diameter and cardiac output. These parameters are conveniently determined by passing the sensor along a ready-sited tube, such as a nasogastric tube, and positioning the sensor accordingly so that it is in close proximity to a blood vessel, such as the descending aorta. The apparatus can thus be readily employed by non-surgical personnel in a convenient manner on the ward, with minimal need for the attendance of physicians to assist in positioning the sensor.

By way of example only, certain embodiments of the invention will now be described with reference to the accompanying drawings, in which:
Figures 1 and 2 illustrate schematically the application of time-gated Doppler ultrasound measurements to the descending aorta using a sensor located in a nasogastric tube;
Figure 3 is a plot illustrating the processing performed by the apparatus used in figures 1 and 2;
Figures 4 and 5 schematically illustrate a method of aligning a Doppler ultrasound sensor located within a nasogastric tube with the descending aorta.

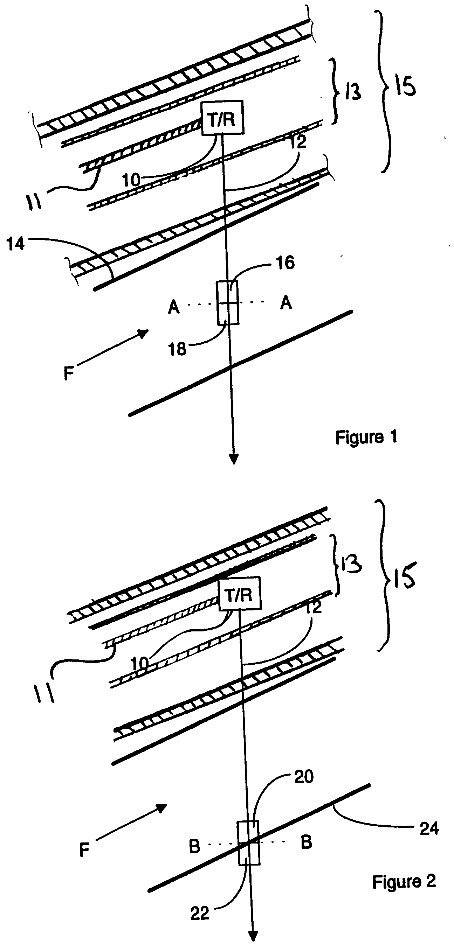

In Figure 1, a sensor 10 comprising an ultrasonic transmitter and an ultrasonic receiver and attached to the end of a rotatable cable 11 passed along a nasogastric tube 13 sited along the oesophagus 15 transmits ultrasonic pulses along path 12 which intersects the descending aorta 14. Note that for simplicity, this and the other Figures only show one transmission path 12 whereas, in practice, it is advantageous to use dual fixed angle paths, as described above. There is a blood flow in a direction of the arrow F in the artery 14. The transmitted ultrasonic pulses are scattered by the blood flow. Sensor 10 acquires ultrasonic signals scattered back along path 12 to sensor 10. As is well known in the art, a single piezoelectric transducer can be used for both transmitting and receiving ultrasonic signals.

The reception of the scattered ultrasonic signals by sensor 10 is time-gated to examine particular locations along path 12. The acquisition of returned signals at sensor 10 is timed so that the returned signals correspond to ultrasonic waves scattered upon the transmitted pulse reaching the desired locations on the transmission path 12. Two time-gated locations 16 and 18 are shown in figure 1. The locations abut one another on the transmission path 12, as indicated by line A-A.

The energy of an acquired signal returning from a given location on path 12 is dependent upon the number of scattering particles in the blood flow at that particular location. In addition, the frequency of the signals returning from a given location will experience a Doppler shift which is dependent upon the vector velocity of the flow at that particular location relative to the reception path (in this case path 12). Thus, for a given location along path 12, there are two specific measurable quantities of the returning signals: energy and Doppler shift. There is also a third, derivable quantity: the product of the energy and the Doppler shift. These three quantities can be regarding as indicator parameters. If an indicator parameter is deduced for each of locations 16 and 18, then the ratio, R, of the indicator parameter values for locations 16 and 18, as shown in figure 1, will be near unity. The ratio R will tend to unity as the extent of locations 16 and 18 along path 12 about line A-A (governed by the time-gating of the sensor 10) tends to zero.

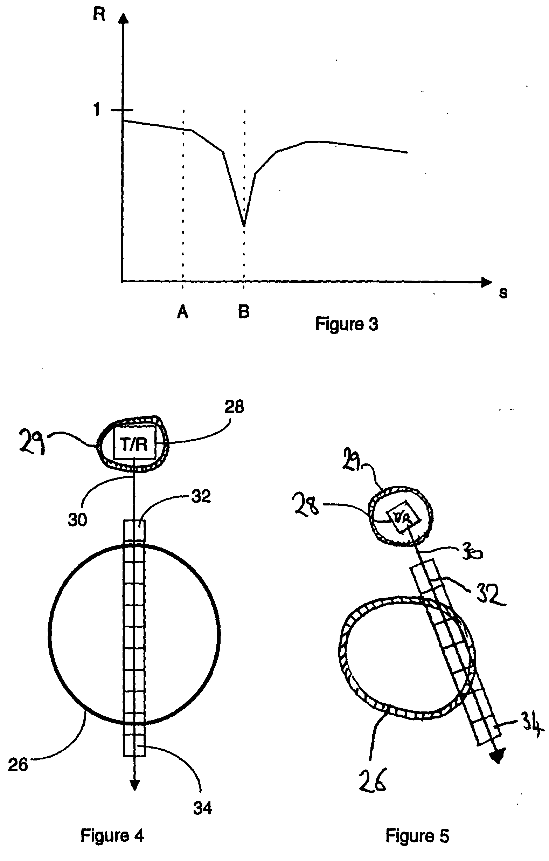

A plot of the ratio R for distance S from the sensor 10 is shown in figure 3. The value of R for the distance along path 12 corresponding to line A-A in figure 1 is indicated A in figure 3.

Figure 2 illustrates the situation where sensor 10 is time-gated to examine two adjacent locations 20 and 22 which meet at a point on path 12 indicated by line B-B. It should be noted also that the intersection of line B-B and path 12 lies on the wall of the descending aorta 24 furthest from sensor 10. It will be appreciated that location 20 lies substantially within the blood flow F and that location 22 lies substantially outside the descending aorta. Thus, the energy scattered from location 20 towards sensor 10 will be considerably different to the amount of energy scattered back from location 22. Similarly, since location 22 is outside the blood flow F, the Doppler shift of signals scattered to sensor 10 from this location will be approximately zero. On the other hand, the signals scattered to sensor 10 from location 20 will exhibit a significant Doppler shift. Considering, therefore, the ratio for any of the aforementioned indicator parameters (energy, Doppler shift or product thereof), it will deviate significantly from unity at point B-B on path 12. Point B-B is illustrated by a notch at B in the plot of R in figure 3.

In operation, the sensor 10 is arranged to time-gate the acquisition of returning signals on path 12 so as to scan a pair of adjacent reception locations along path 12. Effectively, this corresponds to scanning notional line A-A of figure 1 (or notional line B-B of figure 2) along path 12. The sensor is arranged to monitor the ratio R of the values of an indicator parameter from each of the adjacent reception locations. When the ratio R deviates significantly or rapidly from unity, it is determined that the position of a wall of the descending aorta (such as 24) has been encountered. Hence, the separation of the aortic walls may be determined, allowing subsequent calculation of the blood flow rate at this point of the circulation.

It is particularly advantageous to base the ratio R under investigation upon an indicating parameter which has a Doppler shift dependence. Because the Doppler shift falls to zero beyond the blood flow, this means that the interfaces located by monitoring the ratio R are then not the walls of the artery, but the true boundaries of the blood flow therein, hence allowing a yet more accurate measure of flow rate (there may be a region adjacent the arterial walls where there is an insignificant net blood flow).

In making blood flow measurements based on the Doppler shift technique using pulsed-ultrasound, time-gated sensors located in, for example, a nasogastric tube, it is essential to know the position of the transmission path relative to the central axis of the blood vessel under investigation. This information is used in ensuring the correct positioning of the sensor and can be used in the calculation of an overall flow rate for the blood vessel from Doppler-shift-derived velocity measurements at discrete points along the sensor's transmission path within the blood vessel.

Figure 4 illustrates, in cross-section, the descending aorta 26 which is assumed to have a circular cross-section. A sensor 28, located within a nasogastric tube 29 sited along the oesophagus (not shown), is used to investigate the blood flow rate within the descending aorta 26. The sensor 28 comprises a piezoelectric transmitter/receiver which transmits ultrasonic pulses along path 30, and receives returned signals travelling in the opposite direction on the same path. The sensor 28 is time-gated to acquire returning signals which correspond to a sequence of consecutive locations along path 30. Locations 32 and 34 are the terminal locations of this sequence. In figure 4, the transmission path 30 intersects substantially the central axis of the descending aorta 26. Thus, a maximum number of the acquisition locations 32 to 34 will be within the descending aorta 26. Thus, for any of the aforementioned indicator parameters, the cumulative total of the values for all of the locations 32 to 34 will assume a maximum value for the orientation shown in figure 4.

In Figure 5, the transmission path 30 no longer intersects the central axis of the descending aorta 26. Thus, a fewer number of acquisition locations 32 to 34 fall within the descending aorta 26 than in the orientation shown in figure 4. Thus, the cumulative total of the values of a given indicator parameter for acquisition locations 32 to 34 will be lower in the Figure 5 orientation than in the Figure 4 orientation. By rotating sensor 28 using the flexible cable (11 in Figures 1 and 2) to maximise the cumulative total of the values of an indicator parameter totalled across the acquisition locations 32 to 34, misalignment can be minimised.

The minimisation of the cumulative total of an indicator parameter may be achieved manually by allowing a user to monitor the cumulative total of the indicator parameter in question whilst repositioning the sensor 28. Alternatively, the sensor 28 may contain processing circuitry which monitors the cumulative total of the indicator parameter in question as the sensor 28 is repositioned, the processing circuitry noting a maximum value which indicates a minimum misalignment.

In the method described with reference to figures 4 and 5, a consecutive sequence of gates is used. In an alternative arrangement, the sensor 28 could be arranged to acquire returning signals from a single location along path 30 within the descending aorta 26. The previously-defined misalignment could be minimised by examining, as the sensor 28 is repositioned, the variation of a Doppler-shift-based indicator parameter of signals returned from the single location on the path 30. A Doppler-shift-based indicator parameter will have a dependence upon the velocity of the flow at the location sensed. The distribution of velocities within the aortic vessel 26 allows these determinations to be made. The blood flow velocity is highest on the central axis of the vessel 26 and decreases radially to zero at the blood vessel walls (assuming, amongst other things, that the aorta has a circular cross-section).

It will be apparent to one skilled in the art that the apparatus and methods described herein would not be limited in their application to analysis of the descending aorta from an oesophageal position. Thus, other bodily tracts or cavities could be intubated and used for the passage of the transmission and detection means, such that other blood vessels or fluid transport systems within the body could be interrogated.

## Claims

1. Apparatus for remotely locating biological material interfaces, the apparatus comprising means for transmitting a signal into the biological material, means for detecting the signals returned from at least one pair of locations along the path of the transmitted signal, means for determining from the returned signals whether there is an interface between each pair of locations., means for passing at least the transmission and detection means along an exogenous conduit placed within a bodily tract with access to the extracorporeal environment and means for remotely controlling the position of the transmission and detection means within the exogenous conduit.

2. Apparatus according to claim 1 in which the detecting means is capable of detecting signals returned from a plurality of pairs of locations along the transmission path.

3. Apparatus according to claim 2 in which the locations in each pair abut one another.

4. Apparatus according to claim 3 in which the pairs of locations are chosen so that the sweep of the detector across the path of the transmitted signal is essentially continuous.

5. Apparatus according to any preceding claim in which the transmission and detection means are capable both of being moved along the exogenous conduit and of being rotated within the exogenous conduit about a longitudinal axis of the exogenous conduit.

6. Apparatus according to any preceding claim in which the determining means compares the returned signals on the basis of at least one of their energy and frequency.

7. Apparatus according to any preceding claim in which the transmitted signal is an ultrasonic signal.

8. Apparatus according to any preceding claim in which there are provided two paths for conveying signals between the sensing and processing means.

9. Apparatus according to claim 8 in which the two paths for conveying signals comprise a pair of twisted wires.

10. Apparatus according to claim 8 or claim 9 further including isolating means for preventing the passage of potentially damaging signals between the processing means and the sensing means or the test subject.

11. Apparatus according to claim 10 in which the isolating means is a transformer,

12. Apparatus according to any preceding claim in which the sensing means comprises at least one piezoelectric transducer.

13. Apparatus according to any preceding claim in which the sensing means comprises dual transmission and detection transducers with a known, fixed angle between their signal paths.

14. Apparatus according to any preceding claim in which the means for passing the sensing means along the exogenous conduit comprises a flexible cable which, in use, is capable of being rotated about its longitudinal axis without developing substantial elastic twisting forces.

15. Apparatus according to any preceding claim in which the remote control of the sensor position within the exogenous conduit is achieved by means of a rotatable knob connected to the extracorporeal end of the passing means.

16. Apparatus according to any preceding claim in which the exogenous conduit is a nasogastric tube.

17. Apparatus according to claim 16 in which the biological material interface is the interior wall of the descending aorta.

18. A method of remotely locating biological material interfaces, the method comprising transmitting a signal into the biological material using an apparatus according to any preceding claim with its sensing means located in an exogenous conduit placed within a bodily tract with access to the extracorporeal environment, detecting the signals returned from at least one pair of locations along the path of the transmitted signal and determining from the returned signals wither there is an interface between each pair of locations.

19. A method according to claim 18 wherein the biological material interface is the boundary between a blood flow and an artery wall.

20. A method according to claim 18 or claim 19 wherein the transmission and detection means are positioned within the exogenous conduit both by movement along the exogenous conduit and by rotation within the exogenous conduit about a longitudinal axis of the exogenous conduit.

21. A method according to any of claims 18 to 20 wherein the determining step comprises comparing the returned signals on the basis of at least one of their energy and frequency.

22. A method according to any of claims 18 to 21 wherein the transmitted signal is an ultrasonic signal.

23. A method according to any of claims 18 to 22 wherein two biological material interfaces are located, and their separation determined.

24. A method according to claim 23 wherein the boundaries of a fluid flow in a biological vessel are determined such that the fluid flow rate can be determined.

25. A method according to claim 24 wherein the exogenous conduit is a nasogastric tube.

26. A method according to claim 25 wherein the biological material interfaces are the internal walls of the descending aorta, and the fluid flow rate determinable is the rate of blood flow in this vessel.

27. A method of remotely aligning a sensor relative to a biological vessel, the method comprising providing a sensor capable of being passed, using passing means, along an exogenous conduit placed within a bodily tract with access to the extracorporeal environment and which sensor transmits a signal along a path, remotely positioning the sensor such that the path intersects the biological vessel, detecting the signals returned from the biological vessel and determining from the returned signals whether the path intersects substantially the centre of the biological vessel.

28. A method according to claim 27 wherein the sensor is remotely positioned using the passing means by movement along the exogenous conduit and by rotation about a longitudinal axis of the exogenous conduit.

29. A method according to claim 27 or claim 28 wherein the passing means comprises a flexible cable which, in use, is capable of being rotated about its longitudinal axis without developing substantial elastic twisting forces.

30. A method according to any of claims 27 to 29 wherein the signals returning from the biological vessel are detected for several different orientations of the sensor relative to the biological vessel.

31. A method according to claim 30 wherein the detected signals are used to determine the location of the centre of the biological vessel relative to the sensor path and thus permit the sensor to be realigned so that the path intersects substantially the centre of the biological vessel.

32. A method according to any of claims 27 to 31 wherein signals returned from one location on the signal path are detected and the sensor is realigned until, for at least one property, the value thereof for signals returned from the one location is optimised.

33. A method according to any of claims 27 to 31 wherein signals returned from a plurality of locations on the signal path are detected and the sensor is realigned until, for at least one property, the total of the values thereof for signals returned from the plurality of locations is optimised.

34. A method according to any of claims 27 to 33 wherein the determining step comprises analysing the returned signals on the basis of at least one of their energy and frequency.

35. A method according to any of claims 27 to 34 wherein the transmitted signal is an ultrasonic signal.

36. A method according o any of claims 27 to 35 wherein there are used two paths for conveying signals between the sensing and processing means.

37. A method according to claim 36 wherein the two paths for conveying signals comprise a pair of twisted wires.

38. A method according to claim 36 an isolating means is also used for preventing the passage of potentially damaging signals between the processing means and the sensing means or the test subject.

39. A method according to claim 38 in which the isolating means used is a transformer.

40. A method according to any of claims 27 to 39 wherein the sensing means comprises at least one piezoelectric transducer.

41. A method according to any of claims 27 to 40 wherein the sensing means comprises dual transmission and detection transducers with a known, fixed angle between their signal paths.

42. A method according to any of claims 27 to 41 wherein the remote control of the sensor position within the exogenous conduit is achieved by means of a rotatable knob connected to the extracopereal end of the passing means.

43. A method according to any of claims 27 to 42 wherein the exogenous conduit used is a nasogastric tube.

44. A method according to claim 43 wherein the biological vessel is the descending aorta.

45. Apparatus for carrying out the method of any of claims 27 to 44, the apparatus comprising sensing means which transmits a signal along a path, detector means for detecting signals returned from a biological vessel lying in the path of the signal, determining means for determining from the returned signals whether the transmission path intersects substantially the centre of the biological vessel, means for passing at least the transmission and detection means along an exogenous conduit placed within a bodily tract with access to the extracorporeal environment and means for remotely controlling the position of the transmission and detection means within the exogenous conduit.
